# EUROPÄISCHE PATENTSCHRIFT

(11) **EP 0 750 885 B1**
(45) Veröffentlichungstag und Bekanntmachung des Hinweises auf die Patenterteilung: **10.03.2004**
(21) Anmeldenummer: 96107039.8
(22) Anmeldetag: 06.05.1996
(51) Int. Cl.: A61B 17/00

(54) **Gerät zur Anwendung in der Endoskopie und Laparoskopie**
Device to be used in endoscopie and laparoscopie
Appareil pour l'utilisation en endoscopie et en laparoscopie

(30) Priorität: 16.05.1995 AT 83095
(43) Veröffentlichungstag der Anmeldung: 02.01.1997
(73) Patentinhaber: Nycomed Austria GmbH, 4020 Linz (AT)
(72) Erfinder: Scheyer, Mathias, Dr., 6800 Feldkirch (AT)
(74) Vertreter: Plougmann & Vingtoft A/S

(56) Entgegenhaltungen:
- EP-A- 0 557 963
- WO-A-92/06638
- US-A- 5 295 952
- US-A- 5 310 407
- US-A- 5 350 387

## Beschreibung

Die Erfindung betrifft ein Gerät für endoskopische oder laparoskopische Applikation von chirurgischem Material, insbesondere von wundversiegelndem oder wundheilendem Material.

Die Durchführung von endoskopischen oder laparoskopischen Eingriffen erfordert die Verwendung spezieller chirurgischer Instrumente, die an die Verwendung in Trokaren oder Laparoskopen angepaßt sind. Ein besonderes Problem stellt dabei die genaue und sichere Plazierung und Fixierung von chirurgischen Instrumenten und/oder Hilfsmitteln an der gewünschten Stelle im Körper dar (siehe zum Beispiel die US-A-5 295 952 oder die US-A-5 310 407).

Aus EP - A 0 543 499 ist eine Vorrichtung zum Einbringen und Positionieren einer Antiadhäsionsschicht bekannt, wo bei das Antiadhäsionsmaterial mit Hilfe einer aus einer Düse austretenden Flüssigkeit, beispielsweise einer Salzlösung an der gewünschten Stelle fixiert wird. Nachteil dieser Vorrichtung ist, daß das zu applizierende Material vor dem Einbringen in den Körper nur ungenügend an Applikator befestigt werden kann und daher große Geschicklichkeit bei der Handhabung erfordert.

Aufgabe der vorliegenden Erfindung war es daher, ein Gerät für die Anwendung in endoskopischen oder laparoskopischen Eingriffen bereitzustellen, bei dem die sichere Befestigung von chirurgischen Instrumenten oder Hilfsmitteln, insbesondere wundversiegelndem oder wundheilendem Material einerseits und die sichere Handhabbarkeit und genaue Plazierung des Instruments oder Hilfsmittels an der gewünschten Stelle gewährleistet wird.

Gegenstand der Erfindung ist daher ein Gerät für endoskopische oder laparoskopische Applikation von chirurgischem Material, bestehend aus einem Handgriff (1), einem von einem beweglichen Rohr (3) umschlossenen Schaft (2), an dessen Ende einen Verschluß zum Anbringen einer Vorrichtung zum Befestigen des wundheilenden oder wundversiegelnden Materials oder eines chirurgischen Instruments vorgesehen ist, wobei dieser Verschluß aus zwei verschieden langen u-formig verbundenen Schenkeln (6) besteht, und am langeren Schenkel eine Einkerbung (7) vorgesehen ist, und auf der Vorrichtung zum Befestigen des wundheilenden oder wundversiegelnden Materials oder dem chirurgischen Hilfsmittels eine Erhöhung (8) vorgesehen ist, die paßgenau in die Kerbe des längeren Schenkel eingepaßt werden kann.

Dadurch wird eine sichere Fixierung des in den Körper einzubringenden Instruments oder Materials gewährleistet.
Der Begriff chirurgisches Material wird in dieser Anmeldung für chirurgische Instrumente, Hilfsmittel und wundheilendes oder wundversiegelndes Material verwendet.

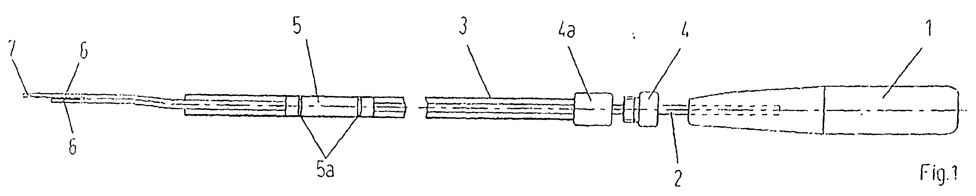

Figur 1 zeigt das erfindungsgemäße Gerät.

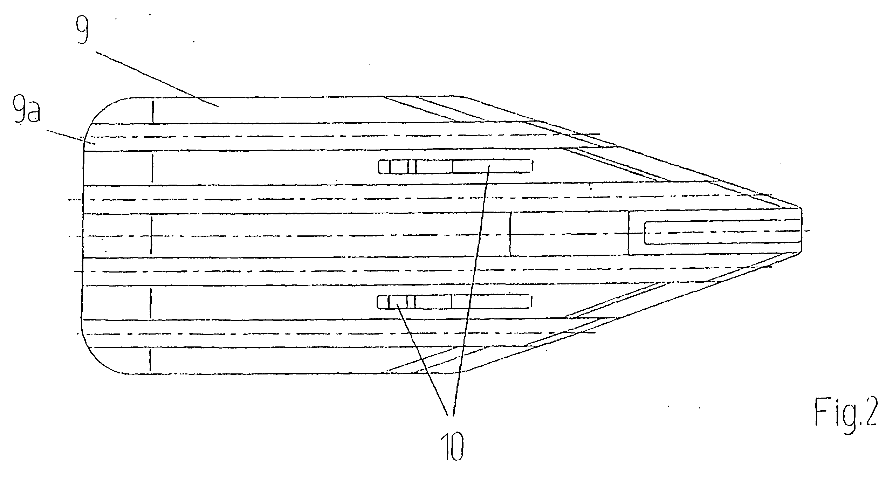

Figur 2 zeigt die Vorrichtung zumBefestigen des wundheilenden Materials im Grundriß.

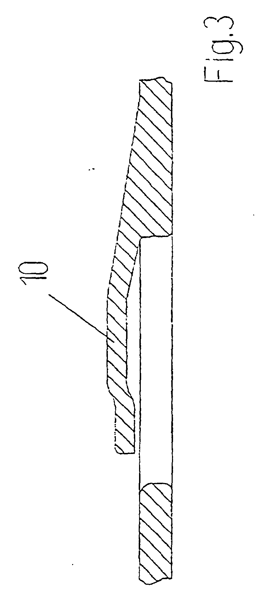

Figur 3 zeigt einen Schnitt durch die Ebene C-C aus Figur 2

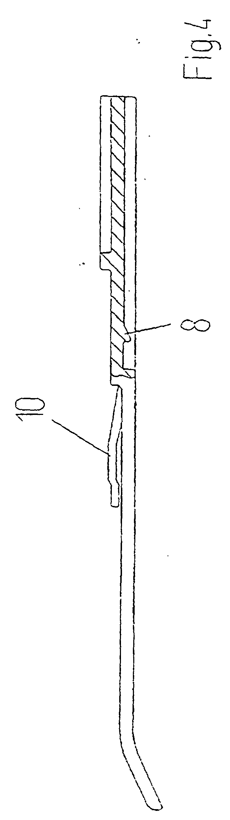

Figur 4 zeigt die Vorrichtung zum Befestigen des wundheilenden oder wundversiegelnden Materials im Aufriß.

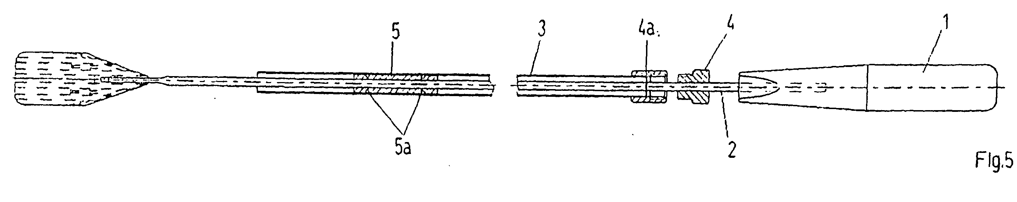

Figur 5 zeigt das Gerät mit der Vorrichtung zum Befestigen des wundheilenden oder wundversiegelnden Materials.

In den Zeichnungen bedeutet (1) den Griff des Gerätes, (2) den daran fixierten Schaft, (3) das bewegliche Rohr, (4) die Verschlußschraube, (4a) die Hülse, (5) das Lager mit dem Dichtungsring (5a, 6) die beiden u-förmig verbundenen verschieden langen Schenkel, (7) die Einkerbung im längeren Schenkel, (8) die Erhöhung an der Vorrichtung zum Befestigen des wundheilenden oder wundversiegelnden Materials, (9 und 9a) die beweglichen Lamellen, und (10) die Klappen zur Befestigung des wundheilenden oder wundversiegelnden Materials.

Das am Schaft (2) befestigte Instrument oder die Vorrichtung zum Befestigen des wundheilenden oder wundversiegelnden Materials wird vor dem Einbringen in den Trokar durch Bewegen des Rohrs (3) in diesem Rohr verborgen und erst an der gewünschten Stelle im Körper durch Zurückziehen des Rohrs (3) freigesetzt. Dadurch kommt das in den Körper einzubringende Instrument oder Material nicht direkt mit dem Trokar in Kontakt, wobei Verschmutzungen beispielsweise durch Blut vermeiden werden und eine Beschädigung des Materials beim Einbringvorgang ausgeschlossen wird. Außerdem ist eine gleichmäßige, unabhängig vom eingebrachten Material rasche Dichtigwirkung mit definierter exzentirscher Lagerung zwischen Wirkungs-und Manipulationsbereich gegeben.

Der Schaft (2) und das Rohr (3) können aus jedem geeigneten Werkstoff, beispielsweise Metall, wie rost- und säurebeständigem Stahl, oder Kunststoff, wie glasfaserverstärktem PSU bestehen. Zu beachten ist außerdem, daß diese Werkstoffe durch die üblich angewandten Sterilisationsmethoden sterilisiert und resterilisiert werden können. Vorteilhaft ist eine möglichst leichte aber stabile Ausführung dieser Teile.

Der Griff (1) besteht vorzugsweise aus Aluminium oder rutschfestem Kunststoff.

Das Rohr (3) wird vorzugsweise mit Hilfe einer Hülse (4a) am Schaft in axialer Richtung beweglich befestigt. Eine am Schaft 2 montierte Verschlußschraube (4) verhindert ein zu weites Zurückziehen des Rohrs. Vorzugsweise ist zur genauen Führung des Rohrs ein Lager (5) mit einem Dichtungsring (5a) vorgesehen, wobei das Lager (5) aus rost- und säurebeständigem Stahl und der Dichtungsring (5a) aus Silikon oder oder anderen sterilisierbaren Kunststoffen bestehen kann

Der Schaft (2) kann vorzugsweise in der Nähe der u-förmig verbundenen Schenkel (6) einen Knick aufweisen. Dadurch wird eine definierte, exzentrische Lage des befestigten chirurgischen Materials oder der Vorichtung zu desen Befestigung erreicht.

Ein weiterer Gegenstand der Erfindung ist die Vorrichtung zum Befestigen eines wundheilendem oder wundversieglendem Material, das aus beweglichen Lamellen (9 und 9a) besteht und an der Innenseite Klappen (10) zur Aufnahme des wundversiegelnden oder wundheilenden Materials vorsieht.

Diese Vorrichtung weist vorzugsweise die Form einer abgerundeten und abgeschrägten Platte auf und kann aus sterilisierbarem Kunststoff, wie Polypropylen oder ähnlichem gefertigt werden und ist vorzugsweise zum einmaligen Gebrauch bestimmt. Die Lamellen (9 und 9a) müssen dabei ausreichend stabil und dennoch biegsam sein um den Anpreßdruck an die gewünschte Stelle genau dosieren zu können und gleichzeitig durch leichtes Abziehen das wundheilende oder wundversiegelnde Material an dieser Stelle zu fixieren.

Sie wird mit der an der Rückseite vorgesehenen Erhöhung (8) in der Einkerbung (7) des Schafts befestigt. Anschließend wird das bewegliche Rohr (3) über die Vorrichtung gezogen, wobei sich die beweglichen Lamellen (9, 9a) konusförmig schließen und das wundversiegelnde oder wundheilende Material einschließen.

Anschließend wird das Gerät in den Trokar eingeführt und an der gewünschten Stelle werden durch Zurückziehen des Rohres die Lamellen entfaltet. Anschließend kann das wundheilende oder wundversiegelnde Material durch leichtes Andrücken und Wegziehen aus seiner Befestigung in den Klappen gelost und an der vorgesehenen Stelle fixiert werden.

## Patentansprüche

1. Gerät für endoskopische oder laparoskopische Applikation von chirurgischem Material, bestehend aus
- einem Handgriff (1),
- einem von einem beweglichen Rohr (3) umschlossenen Schaft (2), an dessen Ende
- ein Verschluss zum Anbringen einer Vorrichtung zum Befestigen des wundheilenden oder wundversiegelnden Materials oder eines chirurgischen Instruments vorgesehen ist,
- wobei dieser Verschluss aus zwei verschieden langen u-förmig verbundenen Schenkeln (6) besteht, und am längeren Schenkel eine Einkerbung (7) vorgesehen ist, und auf der Vorrichtung zum Befestigen des wundheilenden oder wundversiegelnden Materials oder auf dem chirurgischen Material eine Erhöhung (8) vorgesehen ist, die passgenau in die Kerbe des längeren Schenkels eingepasst werden kann, wobei
die Vorrichtung zum Befestigen von wundheilendem oder wundversiegelndem Material besteht aus einer abgerundeten und angeschrägten Platte mit beweglichen Lamellen (9, 9a) an deren Innenseite Klappen (10) zum Befestigen des chirurgischen Materials angebracht sind.

2. Gerät nach Anspruch 1, **dadurch gekennzeichnet, daß** der Schaft 2 in der Nähe der u-förmig verbundenen Schenkel 6 einen Knick aufweist, wodurch eine definierte exzentrische Lagerung zwischen Wirkungs- und Manipulationsbereich erreicht wird.

3. Gerät nach Anspruch 1, **dadurch gekennzeichnet, daß** die Vorrichtung zum Befestigen des wundversiegelndem oder wundheilendem Materials aus beweglichen Lamellen 9, 9a besteht und an der Innenseite Klappen zum Befestigen des wundheilenden oder wundversiegelnden Materials angebracht sind

4. Gerät nach einem der Ansprüche 1 oder 2 **dadurch gekennzeichnet, daß** die Vorrichtung zum Befestigen des wundheilenden oder wundversieglendem Materials die Form einer abgerundeten und angeschrägten Platte hat.

## Claims

1. An appliance for endoscopic or laparoscopic application of surgical material, comprising
- a handle (1)
- a shank (2) received in a movable tube (3), at the end of which shank
- a fastener for positioning a device for fastening wound healing or wound sealing material or a surgical instrument is placed
- the fastener comprising two arms (6) of different lengths interconnected in a U-shape, the longest arm having a notch (7), and the device for fastening the wound healing or wound sealing material or the surgical material having a prominence (8) which fits perfectly into the notch of the longest arm,
the device for fastening the wound healing or wound sealing material comprising a rounded-off and chamfered plate with movable lamellas (9, 9a), at the inner side of which flaps (10) for fastening the surgical material are placed.

2. Appliance according to claim 1, **characterised in that** the shank (2) has a bend close to the two arms (6) which are interconnected in a U-shape, whereby a defined eccentric positioning between the function area and the manipulation area is obtained.

3. Appliance according to claim 1, **characterised in that** the device for fastening the wound sealing or wound healing material is composed of movable lamellas (9, 9a) and that flaps for fastening the wound healing or wound sealing material are placed at the inner side.

4. Appliance according to any of claims 1 or 2, **characterised in that** the device for fastening the wound healing or wound sealing material has the shape of a rounded-off and chamfered plate.

## Revendications

1. Outil pour l'application endoscopique ou laparoscopique d'une matière chirurgicale comprenant
- une poignée (1)
- une queue (2) entourée d'un tube amovible (3), à l'extrémité de laquelle se trouve
- un fermoir pour le positionnement d'un dispositif pour la fixation d'une matière cicatrisante ou scellante pour plaies ou d'un instrument chirurgical
- le fermoir comprenant deux bras (6) de longueurs différentes, qui sont reliés en forme d'U, le bras le plus long ayant une encoche (7), et le dispositif pour la fixation de la matière cicatrisante ou scellante pour plaies ou la matière chirurgicale ayant une proéminence (8) pouvant s'insérer de manière parfaite dans l'encoche du bras le plus long,
le dispositif pour la fixation de la matière cicatrisante ou scellante pour plaies comprenant une plaque arrondie et chanfreinée avec des lamelles amovibles (9, 9a), à la face intérieure desquelles son placés des volets (10) pour la fixation de la matière chirurgicale.

2. Outil selon la revendication 1, characterisé en ce que la queue (2) présente une courbure près des deux bras (6) reliés en forme d'U, ce qui permet d'obtenir une position excentrique entre le domaine de fonctionnement et le domaine de manipulation.

3. Outil selon la revendication 1, characterisé en ce que le dispositif pour la fixation de la matière cicatrisante ou scellante pour plaies est composé de lamelles amovibles (9, 9a) et que les volets pour la fixation de la matière cicatrisante ou scellante pour plaies sont placés à la face intérieure.

4. Outil selon l'une quelconque des revendication 1 ou 2, characterisé en ce que le dispositif pour la fixation de la matière cicatrisante ou scellante pour plaies a la forme d'une plaque arrondie et chanfreinée.
